# EUROPEAN PATENT SPECIFICATION

(11) **EP 3 858 284 B1**
(45) Date of publication and mention of the grant of the patent: **15.10.2025**
(21) Application number: 21154135.4
(22) Date of filing: 28.01.2021
(51) Int. Cl.: A61B 90/11, A61B 90/50, A61B 90/14

(54) **PATIENT STABILIZATION SYSTEM**
PATIENTENSTABILISIERUNGSSYSTEM
SYSTÈME DE STABILISATION DE PATIENT

(30) Priority: 31.01.2020 US 202016778137
(43) Date of publication of application: 04.08.2021
(73) Proprietor: Globus Medical, Inc., Audubon, PA 19403 (US)
(72) Inventor: CLEARY, David, Somerville, MA 02144 (US); TRACY, Steve, Litchfield, NH 03052 (US); JOHNSON, Norbert, North Andover, MA 01845 (US); ZHANG, Kevin, Medford, MA 02155 (US); CAMERON, Hayden, Philadelphia, PA 19129 (US); PINARD, Gary, Manchester, NH 03109 (US)
(74) Representative: Morabito, Sara

(56) References cited:
- EP-A1- 3 662 858
- WO-A1-2008/115151
- WO-A1-2016/185140
- DE-U1- 8 400 384
- US-A- 6 129 319
- US-A1- 2018 132 965
- US-B2- 8 393 588

## Description

### BACKGROUND

Robot assisted medical procedures often require a high degree of precision. Position recognition system are generally used to determine three-dimensional (3D) position of the patient. Surgical systems utilize the determined 3D position to precisely guide movement of instrumentation guided by robotic arms and tools relative to the patient. However, movement between the patient and the instrumentation may reduce the precision of the surgical systems. Traditionally, a patient fixation apparatus (e.g., stereotactic frame) may be used to hold the patient in position. In some cases, the instrumentation is directly coupled to the patient fixation apparatus to minimize movement between the patient and the instrumentation. US2018132965A1 discloses such a system of the prior art. However, some surgical systems include robot arms or tools that support the instrumentation. These robot arm or tools are generally decoupled from the patient fixation device for various reasons such as size, space, convenience, and/or limited availability. As such, movement between the patient and the instrumentation may occur when using these robot arms or tools.

The invention is defined in appended independent claim 1, further embodiments are described in the dependent claims.

### BRIEF DESCRIPTION OF THE DRAWINGS

These drawings illustrate certain aspects of some of the embodiments of the present disclosure and should not be used to limit or define the disclosure.
FIG. 1 is an overhead view of a surgical system having a patient stabilization system and a surgical robot system, according to some embodiments;
FIG. 2 is an overhead view of an alternate embodiment of the surgical system having the patient stabilization system and the surgical robot system, according to some embodiments;
FIG. 3 is a side view of the patient stabilization system anchored to the surface and attached to the patient fixation device, according to some embodiments;
FIG. 4 is a is a perspective view of the patient stabilization system, according to some embodiments;
FIG. 5 is a perspective view of an adaptor for the patient stabilization system, according to some embodiments;
FIG. 6 is a perspective view of another adaptor for the patient stabilization system, according to some embodiments;
FIG. 7 is a cross-section view of the patient stabilization system, according to some embodiments;
FIG. 8 is a cross-sectional view of the locking mechanism of the patient stabilization system, according to some embodiments;
FIGS. 9A and 9B are respective side views of a deployed wheel and a retracted wheel of the plurality of wheels, according to some embodiments; and
FIG. 10 is a cross-sectional view of a dielectric break disposed within the at least one arm of the patient stabilization system, according to some embodiments.

### DETAILED DESCRIPTION

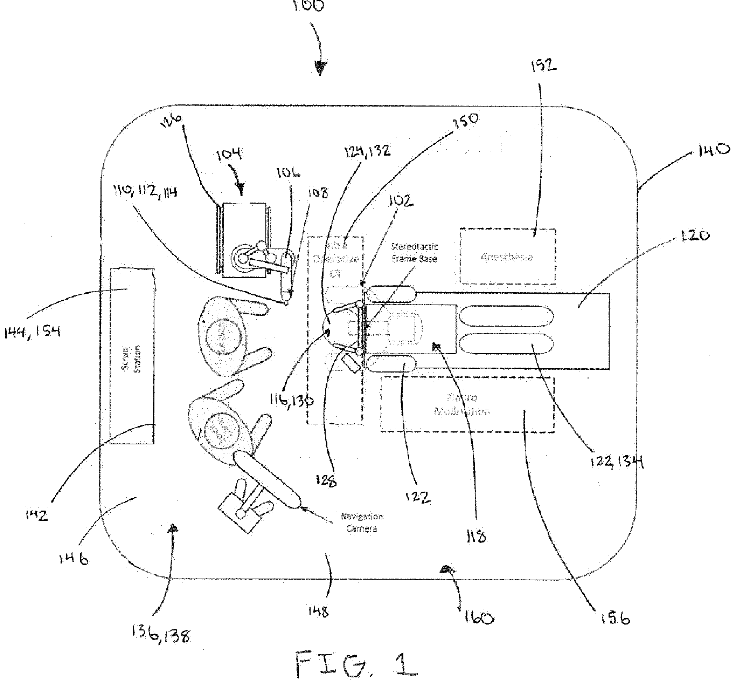

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments. Referring now to the drawings, FIG. 1 illustrates a surgical system 100 having a patient stabilization system 102 and a surgical robot system 104 in accordance with an embodiment. The surgical system 100 may be utilized in at least a portion of a medical procedure (e.g., a cranial procedure). For example, during a medical procedure the surgical robot system 104 may include at least one robot arm 106 having an end-effector 108 that may hold and navigate (e.g., orient and move) a surgical instrument 110 (e.g., a screwdriver, dilator, implant inserter, or the like). In another example, the end-effector 108 may hold and navigate a guide tube 112, which is able to receive and orient the surgical instrument 110 used to perform surgery on the patient 118. As set forth below, the surgical system 100 may employ various devices to aid the surgical robot system 104 in navigating the surgical instruments 110 and the guide tube 112 with respect to targets of surgical interest 116 (e.g., targets within a brain 132 of a patient 118) during the procedure. However, any relative movement between the surgical robot system 104 and the targets of surgical interest 116 during the procedure may at least temporarily mis-position the surgical instrument 110. For example, during the cranial procedure, any number of factors (e.g., the patient coughing, oscillation of an operating table 120 may oscillate, or movement of an extremity 122 of the patient 118) may cause the head 124 of the patient 118 to move with respect to the surgical robot system 104, which may result in relative movement between the surgical robot system 104 and the targets of surgical interest 116 and result in at least temporary mispositioning of the surgical instrument 110. As set forth herein, a robot base 126 of the surgical robot system 104 may be fixed with respect to the targets of surgical interest 116 to minimize relative motion between the robot base 126 and the targets of surgical interest 116.

According to the invention, the surgical system 100 includes a patient fixation apparatus 128 (e.g., stereotactic frame). The patient fixation apparatus 128 may be secured to the patient 118. In particular, the patient fixation apparatus 128 may be secured to the targets of surgical interest 116 (e.g., the brain 132), to another anatomical feature 130 (e.g., the head 124, a leg 134, etc.) of the patient relatively fixed to the targets of surgical interest 116, or some combination thereof. As such, the patient fixation apparatus 128 may be fixed with respect to the targets of surgical interest 116. Moreover, as the patient fixation apparatus 128 may be fixed with respect to the targets of surgical interest 116, fixing the robot base 126 of the surgical robot system 104 with respect to the patient fixation apparatus 128 may also fix the robot base 126 with respect to the targets of surgical interest 116, which may minimize relative movement between the robot base 126 and the targets of surgical interest 116.

In some embodiments, fixing the robot base 126 with respect to the patient fixation apparatus 128 may include independently securing each of the robot base 126 and the patient fixation apparatus 128 to a surface 136 disposed within an operating room 160. The surface 136 may include a floor 138, a wall 140, an exterior 142 of a medical device 144 disposed in the operating room 160, or any other suitable surface 136 disposed within the operating room 160. In some embodiments, the patient fixation apparatus 128 may be secured to a first portion 146 of the surface 136, and the robot base 126 may be secured to a second portion 148 of the surface 136. For example, the patient fixation apparatus 128 may be secured to a portion of the floor 138 (e.g. the first portion 146) disposed beneath the operating table 120, and the robot base 126 may be secured to a portion of the floor 138 (e.g., the second portion 148) offset from the operating table 120. However, as any portion of the surface 136 (e.g., the first portion 146, the second portion 148, etc.) may be fixed with respect to the any other portion of the surface 136, the first portion 146 and second portion 148 may correspond to any suitable portions of the surface 136. In some embodiments, the first portion 146 and the second portion 148 may vary to improve workspace management and flexibility. That is, the robot base 126 and/or the patient fixation apparatus 128 may be secured to different portions of the surface 136 based at least in part on a type of surgery performed, surgeon preferences, other medical equipment present, or any applicable factors.

According to the invention, the patient fixation apparatus 128 is secured to the surface 136 via the patient stabilization system 102. As set forth in detail below, the patient stabilization system 102 may be configured to rigidly attach to the patient fixation apparatus 128, as well as anchor to the surface 136, such that the patient fixation apparatus 128 is fixed with respect to the surface 136. As set forth above, fixing the patient fixation apparatus 128 with respect to the surface 136 may also fix the patient fixation apparatus 128 with respect to the robot base 126, which may minimize relative movement between the robot base 126 and the targets of surgical interest 116. Additionally, fixing the patient apparatus with respect to the surface 136 may prevent general motion of the patient 118 during surgery, which may provide additional benefits during the medical procedure such as maintaining a position of the patient 118 for the other medical devices 144 utilized during the medical procedure. Different medical devices 144 may be positioned with respect to the patient 118 and/or the patient fixation apparatus 128 as desired to facilitate the procedure, such as an intra-operative CT device 150, an anesthesiology station 152, a scrub station 154, and/or a neuro-modulation station 156.

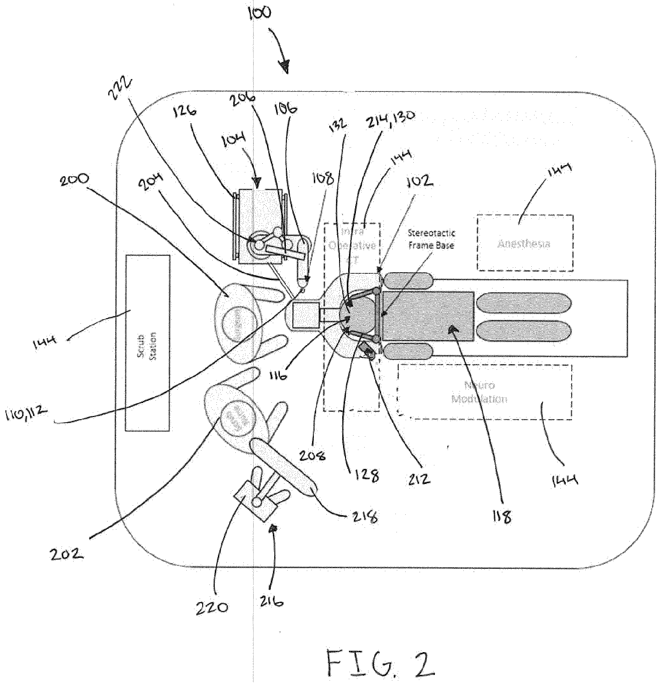

Moreover, in the illustrated embodiment, the patient stabilization system 102 may be anchored to a portion of the surface 136 disposed at least partially beneath the operating table 120. However, the patient stabilization system 102 may be anchored to any portion of the surface 136. For example, the patient stabilization system 102 may be anchored to a portion of the surface 136 disposed adjacent the operating table 120 as shown in FIG. 2, for example. The surface 136 may be a floor 138 of the operating room 160. However, in some embodiments, the surface 136 may be the wall 140, a tabletop, the exterior 142 of the medical device 144, any other suitable surface 136, or some combination thereof.

FIG. 2 illustrates an overhead view of the patient 118, a surgeon 200, other medical personnel 202, and the surgical system 100 having the patient stabilization system 102 and the surgical robot system 104 in accordance with another embodiment. As illustrated, the robot base 126 or another portion of the surgical robot system 104 may be secured directly to the patient stabilization system 102 via a linking arm 204, which may fix the robot base 126 with respect to the patient fixation apparatus 128. As the patient stabilization system 102 is also fixed with respect to the patient fixation apparatus 128, securing the robot base 126 of the surgical robot system 104 to the patient stabilization system 102 may fix the robot base 126 with respect to the patient fixation apparatus 128, which may minimize relative movement between the robot base 126 and the targets of surgical interest 116.

The surgical robot system 104 may include, for example, the robot base 126, the at least one robot arm 106, the linking arm 204, a display 206, the end-effector 108, and one or more tracking markers 208. The robot arm 106 may be movable relative to the robot base 126, responsive to input from a user 210 (e.g., the surgeon 200 or other medical personnel 202), commands received from a processing device, or other methods. The surgical robot system 104 may include a patient tracking device 212 also including the one or more tracking markers 208, which is adapted to be secured directly to the patient 118 (e.g., to a bone 214 of the patient 118). As will be discussed in greater detail below, the tracking markers 208 may be secured to or may be part of a patient fixation apparatus 128 (e.g., stereotactic frame) that is fixed with respect to some anatomical feature 130 of the patient 118.

The surgical robot system 104 may utilize a sensor 216, such as a camera 218. The camera 218 may be positioned on a camera stand 220 that can have any suitable configuration to move, orient, and support the camera 218 in a desired position. The camera 218 may include any suitable camera 218 or cameras, such as one or more cameras (e.g., bifocal or stereophotogrammetric cameras), able to identify, for example, the tracking markers 208. The camera 218 may scan to detect the light that comes from the tracking markers 208 in order to identify and determine the position of the tracking markers 208 in three-dimensions. For example, the tracking markers 208 may be active tracking markers having infrared-emitting markers that are activated by an electrical signal (e.g., infrared light emitting diodes (LEDs)), and/or passive tracking markers 208 having retro-reflective markers that reflect infrared or other light (e.g., they reflect incoming IR radiation into the direction of the incoming light), for example, emitted by illuminators on the camera 218 or other suitable sensor or other device.

In many surgical procedures, one or more targets of surgical interest 116, such as targets within the brain 132 for example, are secured with respect to an external reference frame. For example, stereotactic neurosurgery may use a patient fixation apparatus 128 (e.g., stereotactic frame) that securers to the head 124 or another suitable anatomical feature 130 of the patient 118. Neuronavigation is used to register, e.g., map, targets within the brain 132 based on pre-operative or intraoperative imaging. Using this pre-operative or intraoperative imaging, links and associations can be made between the imaging and the actual anatomical structures (e.g., targets of surgical interest 116) in a surgical environment, and these links and associations can be utilized by the surgical robot system 104 to determine trajectories for the one or more robot arm 106 of the surgical robot system 104 during surgery.

According to some embodiments, the surgical robot system 104 may combine various software and hardware elements to create a system that can be used to plan, register, place and verify the location of the surgical instrument 110 or another medical device 144 with respect to the brain 132 of the patient 118. The surgical robot system 104 may employ a surgical navigation system 222 and planning software to program and control the one or more robot arm 106 of the surgical robot system 104. Additionally, or alternatively, the one or more robot arm 106 may be remotely controlled, such as by nonsterile personnel (e.g., the surgeon 200 or the other medical personnel 202).

In some embodiments, the robot base 126 may be positioned near or next to the patient 118. The robot base 126 may be positioned at any suitable location near the patient 118 depending on the area of the patient 118 undergoing the operation. The camera 218 may be separated from the surgical robot system 104 and positioned near or next to patient 118 in any suitable position that allows the camera 218 to have a direct visual line of sight to the surgical field. In some embodiments, the surgeon 200 may be positioned across from the robot base 126 but is still able to manipulate the end-effector 108 and the display 206. The other medical personnel 202 (e.g., a surgical assistant) may be positioned across from the surgeon 200 again with access to both the end-effector 108 and the display 206. If desired, the locations of the surgeon 200 and the assistant may be reversed. The traditional areas for the anesthesiologist and the nurse or scrub tech may remain unimpeded by the locations of the robot base 126 and the camera 218.

With respect to the other components of the surgical robot system 104, the display 206 can be attached to the robot base 126. In some embodiments, the display 206 can be detached from robot base 126, either within the operating room 160 with the robot base 126, or in a remote location. The end-effector 108 may be coupled to the robot arm 106 and controlled by at least one motor. As set forth above, in some embodiments, end-effector 108 can comprise the guide tube 112, which is able to receive and orient a surgical instrument 110 used to perform surgery on the patient 118. As used herein, the term "end-effector" is used interchangeably with the terms "end-effectuator" and "effectuator element." Although generally shown with the guide tube 112, it will be appreciated that the end-effector 108 may be replaced with any suitable instrumentation suitable for use in surgery. In some embodiments, end-effector 108 can comprise any known structure for effecting the movement of the surgical instrument 110 in a desired manner.

In some embodiments, the surgical robot system 104 is able to control the translation and orientation of the end-effector 108. The surgical robot system 104 is able to move end-effector 108 along x-, y-, and z-axes, for example. The end-effector 108 can be configured for selective rotation about one or more of the x-, y-, and z-axis such that one or more of the Euler Angles (e.g., roll, pitch, and/or yaw) associated with end-effector 108 can be selectively controlled. In some embodiments, selective control of the translation and orientation of end-effector 108 can permit performance of medical procedures with significantly improved accuracy compared to conventional robots that use, for example, robot arms comprising only rotational axes. For example, the surgical robot system 104 may be used to operate on the patient 118, and the one or more robot arm 106 can be positioned above the patient 118, with end-effector 108 selectively angled relative to the z-axis toward the patient 118.

In some embodiments, the position of the surgical instrument 110 may be dynamically updated so that surgical robot system 104 may be aware of the location of the surgical instrument 110 at all times during the procedure. Consequently, in some embodiments, surgical robot system 104 can move the surgical instrument 110 to the desired position quickly without any further assistance from the surgeon 200 (unless the surgeon 200 so desires). In some further embodiments, surgical robot system 104 can be configured to correct the path of the surgical instrument 110 if the surgical instrument 110 strays from the selected, preplanned trajectory. In some embodiments, surgical robot system 104 can be configured to permit stoppage, modification, and/or manual control of the movement of end-effector 108 and/or the surgical instrument 110. Thus, in some embodiments, the surgeon 200 or the other medical personnel may operate the system, and has the option to stop, modify, or manually control the autonomous movement of end-effector 108 and/or the surgical instrument 110.

Moreover, as set forth above, the surgical robot system 104 may include the active tracking markers 208, which may be configured to track the movement of robot arm 106, end-effector 108, patient 118, and/or the surgical instrument 110 in three dimensions. In some embodiments, the active tracking markers 208 can be mounted (or otherwise secured) to the surgical robot system 104, such as to the robot base 126, the one or more arms, and/or the end-effector 108. The active tracking markers 208 can further be mounted (or otherwise secured) to the patient 118. In some embodiments, the active tracking markers 208 can be positioned on the patient 118 spaced apart from the surgical field to reduce the likelihood of being obscured by the surgeon 200, surgical tools, or other parts of the surgical robot system 104. Further, the active tracking markers 208 can be mounted (or otherwise secured) to the surgical instrument 110 (e.g., a screwdriver, dilator, implant inserter, or the like). Thus, the active tracking markers 208 enable each of the marked objects (e.g., the end-effector 108, the patient 118, and the surgical instrument 110) to be tracked by the surgical robot system 104. In some embodiments, surgical robot system 104 can use tracking information collected from each of the marked objects to calculate the orientation and location, for example, of the end-effector 108, the surgical instrument 110 (e.g., positioned in the tube of the end-effector 108), and the relative position of the patient 118.

In some embodiments, pre-operative imaging may be used to identify the targets of surgical interest 116. If desired by the surgeon 200, the surgical robot system 104 will allow for the definition of a reformatted coordinate system. This reformatted coordinate system will have coordinate axes anchored to specific anatomical landmarks on the patient 118, such as the anterior commissure (AC) and posterior commissure (PC) for neurosurgery procedures. In some embodiments, multiple pre-operative exam images (e.g., CT or magnetic resonance (MR) images) may be co-registered such that it is possible to transform coordinates of any given point on the patient 118 to the corresponding point on all other pre-operative exam images.

As used herein, registration is the process of determining the coordinate transformations from one coordinate system to another. For example, in the co-registration of preoperative images, co-registering a CT scan to an MR scan means that it is possible to transform the coordinates of an anatomical point from the CT scan to the corresponding anatomical location in the MR scan. It may also be advantageous to register at least one exam image coordinate system to the coordinate system of a common registration fixture, such as a dynamic reference base (DRB), which may allow the camera 218 to track a position of the patient 118 in the camera 218 space in real-time so that any intraoperative movement of the targets of surgical interest 116 can be detected by the surgical robot system 104 and accounted for by compensatory movement of the surgical robot system 104. However, any intraoperative movement (e.g., relative movement between the targets of surgical interest 116 and the robot base 126) that necessitates compensatory movement may at least temporarily mis-position the surgical devices (e.g., the surgical instrument and/or the guide tube) with respect to a desired trajectory. Thus, to minimize relative motion between the robot base 126 and the targets of surgical interest 116 during the procedure, a robot base 126 of the surgical robot system 104 may be fixed with respect to the targets of surgical interest 116, as set forth above, such that a position and/or orientation of the targets of surgical interest 116 remains substantially constant with respect to the robot base 126 of the surgical robot system 104.

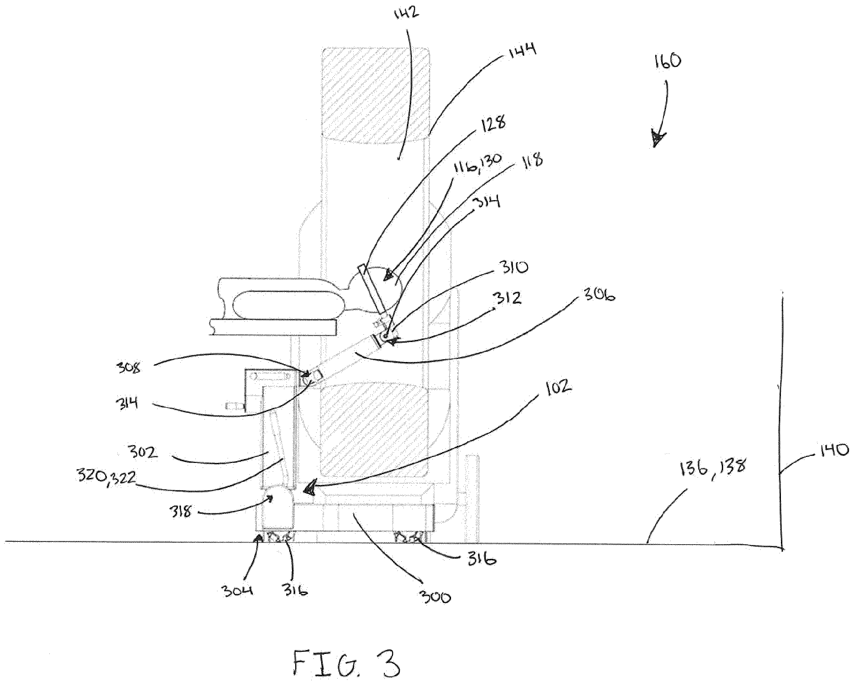

FIG. 3 is a side view of the patient stabilization system 102 anchored to the surface 136 and attached to the patient fixation device in accordance with an embodiment. As set forth above, the patient fixation apparatus 128 may be secured to the patient and/or the targets of surgical interest 116. Thus, as the patient stabilization system 102 is rigidly attached to the patient fixation device as well as anchored to the surface 136, the patient stabilization system 102 may be configured to fix the targets of surgical interest 116 with respect to the surface 136. In some embodiments, fixing the targets of surgical interest 116 to the surface 136, via the patient stabilization system 102, may minimize relative movement between the targets of surgical interest 116 and the robot base 126 of the surgical robot system 104 (e.g., shown in FIG. 2). For example, when the robot base 126 is also be fixed with respect to the surface 136, fixing each of the patient stabilization system 102 and the robot base 126 to the surface 136 may minimize relative movement between the robot base 126 and the targets of surgical interest 116.

According to the invention, the patient stabilization system 102 includes a stabilization base 300. As discussed in further detail below, the stabilization base 300 is configured to anchor the patient stabilization system 102 to the surface 136. In the illustrated embodiment, the surface 136 is the floor 138 of the operating room 160. However, in some embodiments, the surface 136 may be the floor 138 of the operating room 160, the wall 140 of the operating room 160, the exterior 142 of the medical device 144, or some combination thereof. The patient stabilization system 102 also includes a column portion 302 extending upwards from a stabilization base 300. The column portion 302 may extend upwards from any portion of the stabilization base 300. However, in the illustrated embodiment, the column portion 302 extends vertically upward from a portion of the stabilization base 300 adjacent a first end 304 of the stabilization base 300. Moreover, the patient stabilization system 102 includes the at least one stabilization arm 306. A proximal end 308 of the at least one stabilization arm 306 is attached to the column portion 302. In some embodiments not according to the invention, the proximal portion of the at least one stabilization arm 306 may be attached to another portion (e.g., the stabilization base 300) of the patient stabilization system 102. An adaptor 310 is configured to attach to a distal portion 312 of the at least one stabilization arm 306. The adaptor 310 is further configured to attach to the patient fixation apparatus 128. As such, the patient stabilization system 102 may be secured to the patient fixation apparatus 128 via the adaptor 310. In some example, the distal portion 312 of the at least one stabilization arm 306 is configured to attach directly to the patient fixation apparatus 128. In some examples, the at least one stabilization arm 306 and/or the adaptor 310 may be configured to directly attach to an anatomical feature 130 of the patient 118 (e.g., the targets of surgical interest 116).

In some embodiments, the patient stabilization system 102 includes a secured (e.g., anchored) state and an unsecured state. The patient stabilization system 102 may be configured to fix the robot base 126 with respect to the targets of surgical interest 116 while in the secured state. Thus, according to the invention, the patient stabilization is configured to anchor to the surface 136 in the secured state. Further, placing the patient stabilization system 102 in the secured state may include restraining rotation of at least one pivot joint 314 disposed between at least two of the stabilization base 300, the column portion 302, the at least one stabilization arm 306, the adaptor 310, or some combination thereof, such that the patient stabilization system 102 is rigid in the secured state. In the unsecured state, the at least one pivot joint 314 may rotate and/or pivot such features (e.g., the stabilization base 300, the column portion 302, the at least one stabilization arm 306, and/or the adaptor 310) of the patient stabilization system 102 may move with respect to each other. For example, prior to attaching the patient stabilization system 102 to the patient fixation apparatus 128, the at least one pivot joint 314 of the at least one stabilization arm 306 and the adaptor 310 may be loosened to permit the adaptor 310 to be repositioned for attachment to the patient fixation apparatus 128. Further, in some embodiments, the patient stabilization system 102 may be configured to move freely along the surface 136 (e.g., the floor 138 of the operating room 160) disposed within the operating room 160 via a plurality of wheels 316 316, mounted to the stabilization base 300 of the patient stabilization system 102, while in the unsecured state. The patient stabilization system 102 may be placed in the unsecured state before, during, or after the procedure to relocate the patient stabilization system 102 within the operating room 160. Relocating the patient stabilization system 102 may provide additional space for the surgeon 200 (e.g., shown in FIG. 2) and/or other medical devices 144 disposed within the operating room 160 proximate the patient 118.

According to the invention, the patient stabilization system 102 includes a locking mechanism 318 configured to selectively restrain movement of the stabilization base 300 (e.g., anchor the stabilization base 300) with respect to the surface 136 of the operating room 160. As such, the locking mechanism 318 is configured to transition at least a portion of the patient stabilization system 102 between the secured state and the unsecured state. In some embodiments, the locking mechanism 318 includes a lever 320 configured to actuate the locking mechanism 318 between the secured state and the unsecured state. However, any suitable trigger device 322 may be used to actuate the locking mechanism 318. For example, the locking mechanism 318 may include an electronic switch configured to activate a motor or another suitable driving mechanism to actuate the locking mechanism 318 to anchor the stabilization base 300 to the surface 136 in the secured state and release the stabilization base 300 to move along the floor 138 in the unsecured state.

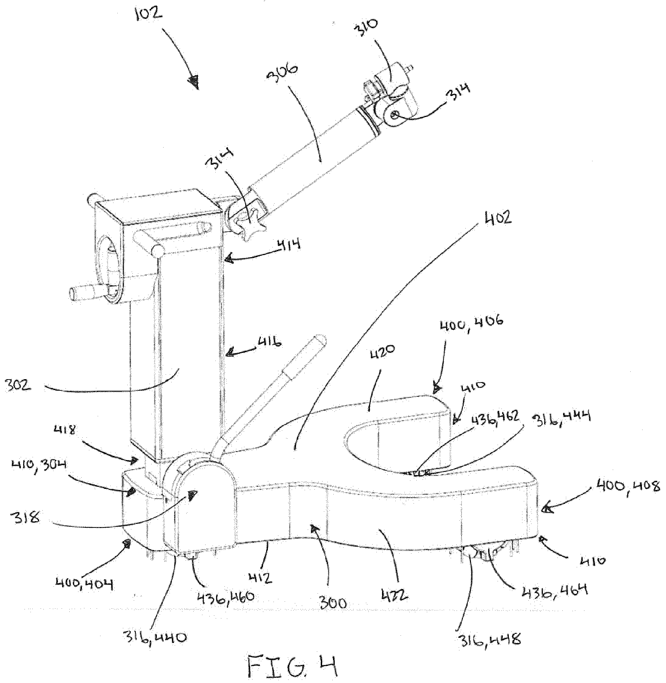

FIG. 4 is a perspective view of the patient stabilization system 102 in accordance with another embodiment. As set forth above, the patient stabilization system 102 may include the stabilization base 300, the plurality of wheels 316, the column portion 302, the at least one stabilization arm 306, the adaptor 310, the locking mechanism 318, or some combination thereof. In some embodiments, the stabilization base 300 includes a plurality of branches 400 extending out from a central portion 402 of the stabilization base 300. For example, in the illustrated embodiment, the stabilization base 300 includes a first branch 404, a second branch 406, and a third branch 408. A distal end 410 of the first branch 404 may form the first end 304 of the stabilization base 300. The column portion 302 may extend upwards from the first branch 404 proximate the first end 304 of the stabilization base 300. However, the column portion 302 may extend upwards from any portion of the stabilization base 300 (e.g., the central portion 402, the first branch 404, the second branch 406, or the third branch 408). In some embodiments, the stabilization base 300 may include any number of branches. In some embodiments, the stabilization base 300 may instead include a polygonal shape (e.g., triangle, square, hexagon, etc.), a circular shape, an oval shape, a non-uniform shape, or any other suitable shape for interfacing with the column portion 302 and the plurality of wheels 316.

In some embodiments, the plurality of wheels 316 is configured to mount to a bottom portion 412 of the stabilization base 300. In the illustrated embodiment, the plurality of wheels 316 comprises three wheels (e.g., a first wheel 440, a second wheel 444, and a third wheel 448). Each of the three wheels may be configured to mount to a separate branch of the stabilization base 300. For example, the first wheel 440 may be configured to mount to the first branch 404, the second wheel 444 may be configured to mount to the second branch 406, and the third wheel 448 may be configured to mount to the third branch 408. In some embodiments, each wheel of the plurality of wheels 316 may be configured to mount to a portion of its respective branch proximate respective distal ends 410 of each of the plurality of branches 400. Attaching the wheel proximate the respective distal ends 410 of the plurality of branches 400 may provide a wider wheelbase for the patient stabilization system 102, thereby, increasing the stability of the patient stabilization system 102. Further, the plurality of wheels 316 having three wheels may increase the stability of the patient stabilization system 102 by having a defined plane and eliminating any nondeterministic rocking that may occur with more or less than three wheels.

In some embodiments, the plurality of wheels 316 may include any number of wheels. For example, the plurality of wheels 316 may include four wheels. In one example, the stabilization base 300 may include four branches such that each wheel corresponds to a separate respective branch. However, in some embodiments, the stabilization base 300 may include fewer than four branches such that at least one branch may have more than one wheels attached thereto. In another example, the stabilization base 300 includes a rectangular shape. In this example, the plurality of wheels 316 may be distributed on the bottom portion 412 of the stabilization base 300 in any suitable pattern.

Further, in some embodiments, the patient stabilization system 102 includes a plurality of support poles 436. In some embodiments, the plurality of support poles 436 includes at least three support poles. Each support pole may be disposed proximate a different wheel of the plurality of wheels 316. For example, at least one first support pole 460 may be disposed proximate the first wheel 440, at least one second support pole 462 may be disposed proximate a second wheel 444, and at least one third support pole 464 may be disposed proximate a third wheel 448

The stabilization base 300 may be configured to support the column portion 302 and the at least one stabilization arm 306 attached to the column portion 302. In some embodiments, the at least one stabilization arm 306 is attached proximate a top portion 414 of the column portion 302. However, in some embodiments, the at least one stabilization arm 306 may be attached to a central portion 416 or lower portion 418 of the column portion 302. Further, in some examples not according to the invention, the at least one stabilization arm 306 may be configured to attached to a top portion 420 or a side portion 422 of the stabilization base 300. In the illustrated embodiment, the at least one stabilization arm 306 is attached to the top portion 414 of the column portion 302 via the at least one pivot joint 314. In some embodiments, the at least one pivot joint 314 may include a hirth joint. However, the patient stabilization system 102 may include any suitable type of pivot joint 314. The at least one pivot joint 314 may be configured to tighten in the secured state to provide a rigid connection between the at least one stabilization arm 306 and the column portion 302. Moreover, the pivot joint 314 may also be configured to loosen in the unsecured state to permit repositioning of the at least one stabilization arm 306 with respect to the column portion 302.

In some examples not according to the invention, the at least one stabilization arm 306 is rigidly secured to the column portion 302 without the at least one pivot joint 314. Moreover, in some embodiments, the at least one stabilization arm 306 may comprise a plurality of sections. For example, the at least one stabilization arm 306 may include a first section and a second section. The first section may be attached to the second section via the at least one pivot joint 314. As such, in the unsecured state, the first section of the at least one stabilization arm 306 may be repositioned with respect to the second section. Further, in the secured state the first section may be rigidly attached to the second section such that the first section is fixed with respect to the second section.

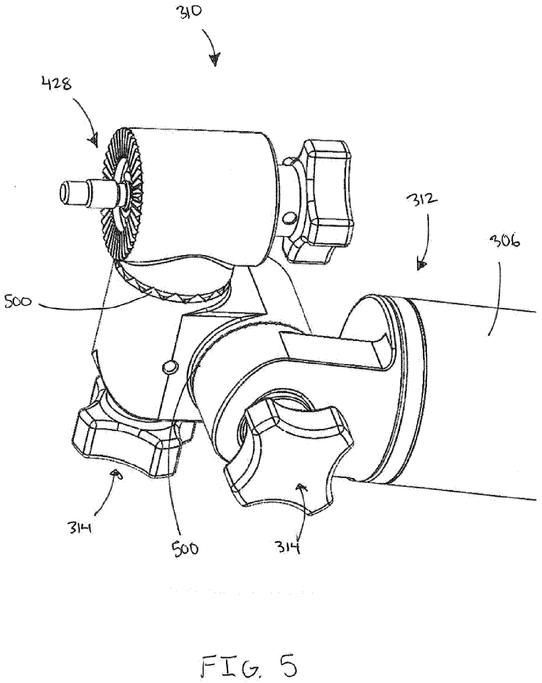

FIG. 5 is a perspective view of the adaptor 310 in accordance with another embodiment. As set forth above, the adaptor 310 may be configured to attach to the distal portion 312 of the at least one stabilization arm 306. The adaptor 310 may further be configured to attach to the patient fixation apparatus 128 as shown in FIG. 3, for example. The adaptor 310 may include a connector interface 428 configured to attach the adaptor 310 to the patient fixation apparatus 128. Further, as each surgeon and/or hospital may have varying preferences or procedure standards, the interface connector may be configured to attach to multiple types of patient fixation apparatuses 128.

In some embodiments, the adaptor 310 also includes the at least one pivot joint 314. As set forth above, the at least one pivot joint 314 may include a hirth joint. The hirth joint may include interlocking teeth 500 configured to provide rigid, zero or minimal backlash, substantially slip-free joint that locks the at least one pivot joint 314 to keep the at least one stabilization arm 306 from being re-positioned with respect to the patient fixation apparatus 128 while the patient stabilization system 102 is in the secured state. In the unsecured state, the at least one pivot joint 314 may be unclamped to permit repositioning of the at least one stabilization arm 306 with respect to the patient fixation apparatus 128.

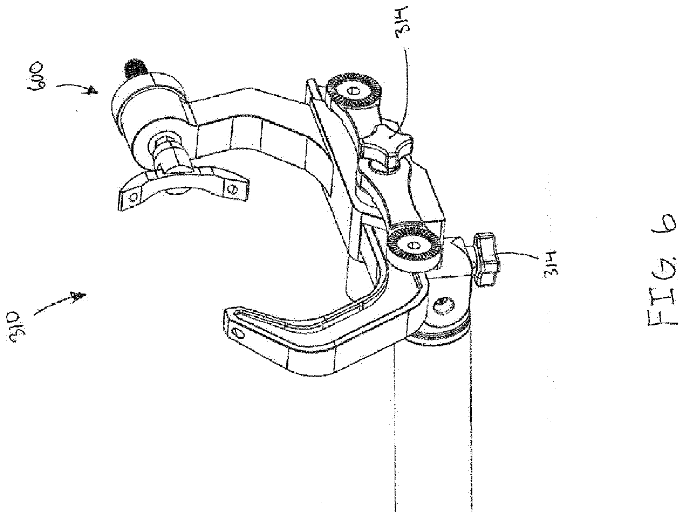

FIG. 6 is a perspective view of another adaptor 310 in accordance with another embodiment. In the illustrated embodiment, the adaptor 310 includes a plurality of pivot joints 314. Additional pivot joint 314 may be configured to provide attachment points for an articulated arm 600. The articulated arm 600 may be utilized for certain procedures or with certain patient fixation apparatuses 128 (e.g., shown in FIG. 3).

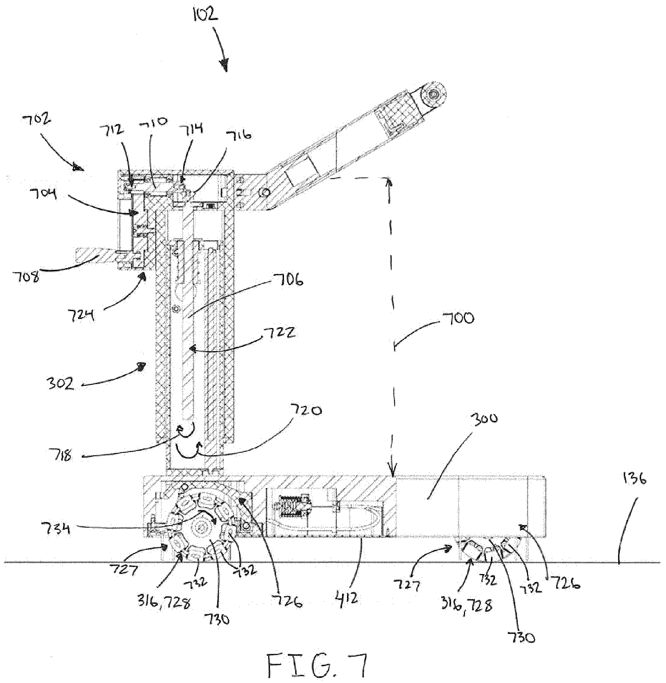

FIG. 7 illustrates a cross-section view of the patient stabilization system 102 in accordance with another embodiment. In some embodiments, the column portion 302 may be an adjustable column that may permit adjustments to a height 700 of the column portion 302. Adjusting the height 700 of the column portion 302 may provide benefits for space management proximate the patient 118 (e.g., shown in FIG. 3) during the procedure. Further, adjusting the height 700 of the column may provide an additional degree of freedom for the patient stabilization system 102, which may aid in attaching the patient stabilization system 102 to the patient fixation apparatus 128 (e.g., shown in FIG. 3).

According to the invention, the patient stabilization system 102 includes a column adjustment device 702 configured to adjust the height 700 of the column portion 302. The column adjustment device 702 may include a hand crank 704. In some embodiments, any suitable device may be used in place of the hand crank 704 or in combination with the hand crank 704 to adjust the height 700 of the column portion 302. Moreover, the hand crank 704 may include any suitable configuration for driving an internal lead screw 706. In the illustrated embodiment, the hand crank 704 includes a handle 708, a cog shaft spindle 710, and the internal lead screw 706. The handle 708 may be rigidly attached to a first end 712 of the cog shaft spindle 710 such that rotation of the handle 708 drives rotation of the cog shaft spindle 710. A second end 714 of the cog shaft spindle 710 may be configured to interface with gear portion 716 of the internal lead screw 706. In particular, the cog shaft spindle 710 may be configured to transfer torque from the rotation of the cog shaft spindle 710 to the internal lead screw 706 via the interface to drive rotation of the internal lead screw 706. Rotation of the internal lead screw 706 in a first direction 718 may increase the height 700 of the column portion 302, and rotation of the internal lead screw 706 in a second direction 720 may decrease the height 700 of the column portion 302. In some embodiments, the internal lead screw 706 includes a pitch 722 configured to restrain back driving of the internal lead screw 706. Restraining back driving may prevent unintended adjustments to the height 700 of the column portion 302. As such, the hand crank 704 may be the only feature of the patient stabilization system 102 configured to adjust the height 700 of the column portion 302. In some embodiments, the hand crank 704 may include a hand crank lock 724 configured to restrain rotation of the hand crank 704 while in the secured state (e.g., during the procedure) such that accidental contact with the hand crank 704 may not adjust the height 700 of the column portion 302 during the procedure.

In some embodiments, the patient stabilization system 102 includes the plurality of wheels 316 configured to mount within respective wheel modules of a plurality of wheel modules 726. The plurality of wheel modules 726 may be mounted, coupled, or otherwise attached to the stabilization base 300 of the patient stabilization system 102. In some embodiments, the plurality of wheel modules 726 is configured to extend into the bottom portion 412 of the stabilization base 300. The plurality of wheels 316 may be at least partially disposed within the plurality of wheel modules 726. A lower portion 727 of each or the plurality of wheels 316 may extend out from a bottom portion 412 of the respective wheel modules 726 and/or stabilization base 300 such that the wheels contact the surface 136 and support the stabilization base 300. In some embodiments, the plurality of wheels 316 include omni wheels 728. The omni wheels 728 may provide multiple degrees of freedom for the plurality of wheels 316. Each omni wheels 728 may include at least one main wheel 730 with a plurality of rollers 732 disposed around the circumference of the wheel. The plurality of rollers 732 may be disposed perpendicular to the direction of rotation 734 of the at least one main wheel 730.

Further, as set forth above in FIG. 4, the patient stabilization system 102 includes the plurality of support poles 436. In some embodiments, the plurality of support poles 436 includes at least three support poles. Each support pole may be disposed proximate a different wheel of the plurality of wheels 316. For example, at least one first support pole 460 may be disposed proximate the first wheel 440, at least one second support pole 462 may be disposed proximate the second wheel 444, and at least one third support pole 464 may be disposed proximate the third wheel 448.

Moreover, as set forth above, the patient stabilization system 102 may include omni wheels 728 having the at least one main wheel 730 configured to rotate as well as a plurality of rollers 732 configured to rotate perpendicular to the direction of rotation of the at least one main wheel 730. As such, preventing rotation of the at least one main wheel 730 may not effectively prevent movement of the patient stabilization system 102 as the plurality of rollers 732 may still move the wheel. Consequently, to anchor the patient stabilization system 102, the locking mechanism 318 may be configured to retract the plurality of wheels 316 in a direction toward the plurality of wheel modules 726 such that the plurality of support poles 436 (e.g., shown in FIG. 4) may contact the surface 136 and support the stabilization base 300 of the patient stabilization system 102. The plurality of support poles 436 may prevent movement of the patient stabilization system 102 with respect to the surface 136; thereby, anchoring the patient stabilization system 102.

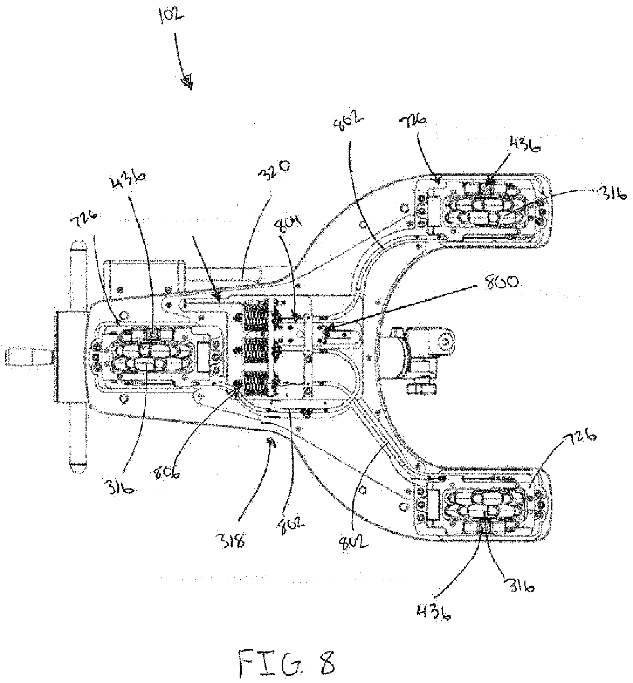

FIG. 8 illustrates a cross-sectional view of the locking mechanism 318 in accordance with another embodiment. As set forth above, the locking mechanism 318 may be configured to anchor the patient stabilization system 102 in the secured state by retracting the plurality of wheels 316 such that the support poles 436 contact the surface 136 and support the patient stabilization system 102. Further, the locking mechanism 318 may be configured to deploy the plurality of wheels 316 in the unsecured state such that the patient stabilization system 102 may move along the surface 136 (e.g., shown in FIG. 3).

As set forth above, the locking mechanism 318 may include the lever 320. In some embodiments, the lever 320 may be configured to move to retract and/or deploy the plurality of wheels 316. The lever 320 may be mechanically coupled to a central linear stage 800 of the locking mechanism 318 such that movement of the lever 320 may actuate the central linear stage 800. The central linear stage 800 may be disposed within the stabilization base 300. In some embodiments, each wheel of the plurality of wheels 316 and/or wheel module of the plurality of wheel modules 726 are coupled to the central linear stage 800 via respective cam links 802. As the central linear stage 800 actuates, based on movement of the lever 320, control cables 804 connected to the central linear stage 800 may be configured to actuate the cam links 802 simultaneously to retract and/or deploy each wheel of the plurality of wheels 316.

Moreover, in some embodiments, the locking mechanism 318 includes a self-locking control mechanism 806 configured to self-lock through a stroke of the lever 320 such that the locking mechanism 318 may not be back driven from forces on the patient stabilization system 102. Thus, in some embodiments, the lever 320 may be the only feature configured to retract and/or deploy the plurality of wheels 316.

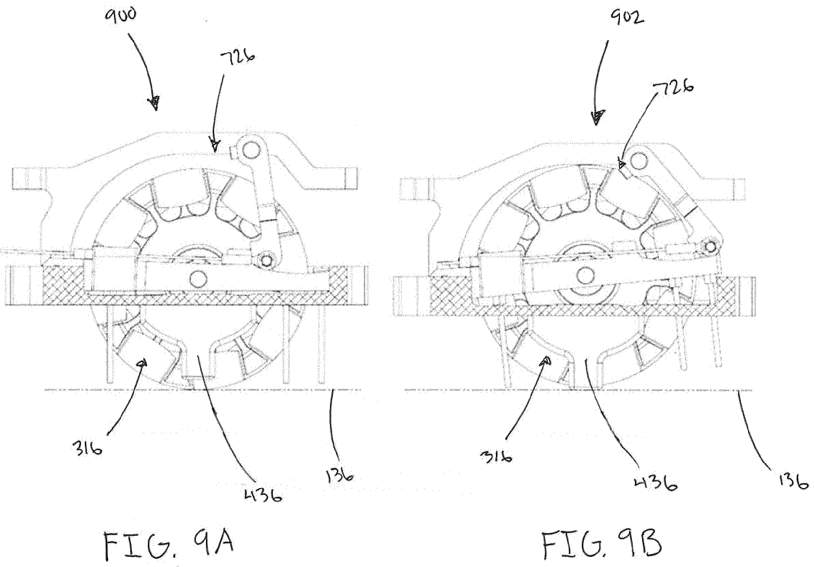

FIGS. 9A and 9B illustrate respective side view of a deployed wheel 900 and a retracted wheel 902 of the plurality of wheels 316. In some embodiments, the lever 320 of the locking mechanism 318 (e.g., shown in FIG. 3) may be moved to a first position to place the patient stabilization system 102 in the unsecured state. Moving the lever 320 to the first position may actuate the locking mechanism 318 to deploy each wheel of the plurality of wheels 316 as shown in FIG. 9A, for example. In the unsecured state, the patient stabilization system 102 may freely move along the surface 136.

In some embodiments, the lever 320 of the locking mechanism 318 may be moved to a second position to place the patient stabilization system 102 in the secured stage. Moving the lever 320 to the second position may actuate the locking mechanism 318 to retract each wheel of the plurality of wheels 316 into their respective wheel modules 726 as shown in FIG. 9B, for example. As the plurality of wheels 316 retract, the plurality of support poles 436 may contact the surface 136. The plurality of support poles 436 may extend downward from the stabilization base 300 to support the patient stabilization system 102 in the secure state. Contact between the plurality of support poles 436 and the surface 136 may restrain or prevent movement of the stabilization base 300 with respect to the surface 136. As such, retracting the plurality of wheels 316 to engage the plurality of poles with the surface 136 may anchor the patient stabilization system 102 to the surface 136. As set forth above in FIG. 3, anchoring the patient stabilization system 102 to the surface 136 may fix the targets of surgical interest 116 with respect to the robot base 126 to minimize relative movement between the surgical robot system 104 and the targets of surgical interest 116.

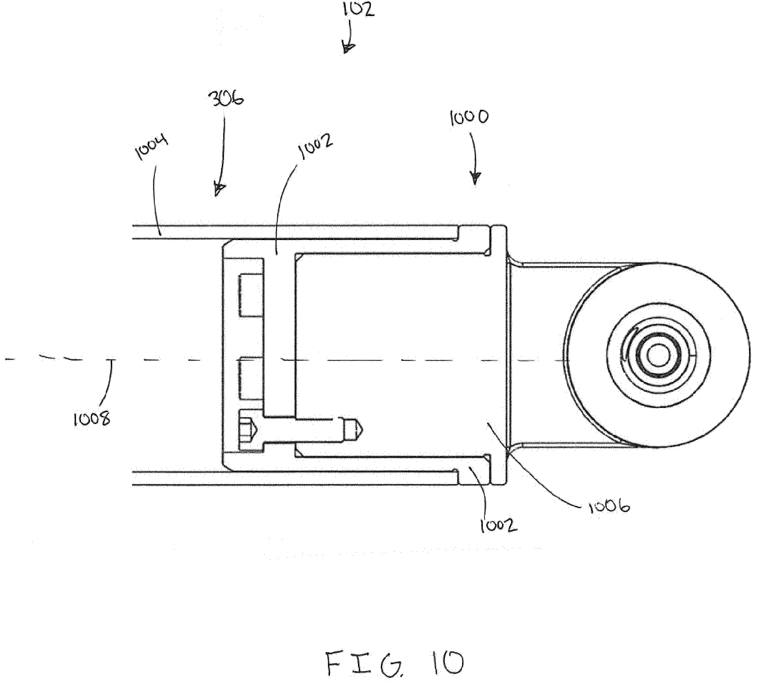

FIG. 10 is a cross-sectional view of a dielectric break 1000 disposed within the at least one stabilization arm 306 of the patient stabilization system 102. In some embodiments, the patient stabilization system 102 includes the dielectric break 1000 to prevent the patient from electrically grounding through the patient stabilization system 102. The dielectric break 1000 may include a non-conductive plug 1002 secured between adjacent portions (e.g., a first arm portion 1004 and a second arm portion 1006) of the at least one stabilization arm 306. The non-conductive plug 1002 may prevent the first arm portion 1004 from contacting the second portion 148. The non-conductive plug 1002 may be rigidly attached to the both the first arm portion 1004 and the second arm portion 1006 such that the first arm portion 1004 may be fixed with respect to the second arm portion 1006. In some embodiments, the non-conductive plug 1002 may be epoxied to the first arm portion 1004 and the second arm portion 1006. However, any suitable attachment mechanism may be utilized to rigidly attach the non-conduction plug to the first arm portion 1004 and the second arm portion 1006. Moreover, in some embodiments, at least a portion of the non-conductive plug 1002 may be disposed radially interior the first arm portion 1004 and radially exterior the second arm portion 1006 of the at least one stabilization arm 306 with respect to a central axis 1008 of the at least one stabilization arm 306.

In the above description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belong. It will be further understood that terms, such as those defined in commonly used dictionaries, should be interpreted as having a meaning that is consistent with their meaning in the context of this specification and the relevant art and will not be interpreted in an idealized or overly formal sense unless expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof.

Although several embodiments of inventive concepts have been disclosed in the foregoing specification, it is understood that many modifications and other embodiments of inventive concepts will come to mind to which inventive concepts pertain, having the benefit of teachings presented in the foregoing description and associated drawings. It is thus understood that inventive concepts are not limited to the specific embodiments disclosed hereinabove, and that many modifications and other embodiments are intended to be included within the scope of the appended claims. It is further envisioned that features from one embodiment may be combined or used with the features from a different embodiment(s) described herein. Moreover, although specific terms are employed herein, as well as in the claims which follow, they are used only in a generic and descriptive sense, and not for the purposes of limiting the described inventive concepts, nor the claims which follow. Various features and/or potential advantages of inventive concepts are set forth in the following claims.

## Claims

1. A surgical system (100) comprising a patient stabilization system (102), the patient stabilization system comprises:
a stabilization base (300);
a plurality of wheels (316) mounted to the stabilization base, wherein the plurality of wheels is configured to move the stabilization base along a surface (136);
a locking mechanism (318) configured to selectively restrain movement of the stabilization base with respect to the surface;
a column portion (302) extending upwards from the stabilization base, the column portion including a column adjustment device (702) configured to adjust a height of the column portion ;
at least one arm (306), wherein the arm comprises a plurality of pivot joints (314), and wherein a proximal portion (308) of the at least one arm is coupled to the column portion via one pivot joint; and
an adaptor (310) coupled to a distal portion (312) of the at least one arm via another pivot joint, wherein the adaptor is configured to couple to a patient fixation apparatus (128) secured to an anatomical feature (130) of a patient (118), wherein the patient stabilization system is configured to anchor the patient fixation apparatus to the surface in a secured state,
wherein the locking mechanism includes a trigger device (322) to actuate the locking mechanism (318) between the secured state and an unsecured state,
wherein the surgical system (100) comprises the patient fixation apparatus (128) and wherein the stabilization base (300), column portion (302) and arm (306) are positioned between the surface (136) and the anatomical feature (130) of the patient (118).

2. The surgical system of claim 1, wherein each wheel of the plurality of wheels is mounted in a respective wheel module (726) of a plurality of wheel modules, and wherein the plurality of wheel modules are mounted to the stabilization base.

3. The surgical system of claim 1, wherein the plurality of wheels comprises omni wheels (728).

4. The surgical system of claim 1, wherein the locking mechanism is configured to retract each wheel of the plurality of wheels into a respective wheel module in the secured state.

5. The surgical system of claim 1, wherein the stabilization base comprises a plurality of support poles (436) extending downward from the stabilization base, wherein the plurality of support poles is configured to support the stabilization base in response to retracting each wheel of the plurality of wheels into a respective wheel module.

6. The surgical system of claim 5, wherein the plurality of support poles comprises three support poles, and wherein each of the three support poles are disposed proximate a different wheel of the plurality of wheels.

7. The surgical system of claim 5, wherein contact between the plurality of support poles and the surface is configured to restrain movement of the stabilization base with respect to the surface.

8. The surgical system of claim 1, wherein the column adjustment device comprises a hand crank (704).

9. The surgical system of claim 8, wherein the hand crank comprises an internal lead screw (706), and wherein the internal lead screw comprises a pitch configured to restrain back driving of the internal lead screw.

10. The surgical system of claim 1, wherein the at least one of the pivot joints comprises a hirth joint.

11. The surgical system of claim 1, wherein the surface comprises a floor of an operating room.

## Patentansprüche

1. Chirurgisches System (100) mit einem Patientenstabilisierungssystem (102), wobei das Patientenstabilisierungssystem aufweist:
- eine Stabilisierungsbasis (300);
- eine Mehrzahl von Rädern (316), die an der Stabilisierungsbasis montiert ist, wobei die Mehrzahl von Rädern eingerichtet ist, um die Stabilisierungsbasis entlang einer Oberfläche (136) zu bewegen;
- einen Verriegelungsmechanismus (318), der eingerichtet ist, um eine Bewegung der Stabilisierungsbasis in Bezug auf die Oberfläche selektiv einzuschränken;
- einen Säulenabschnitt (302), der sich von der Stabilisierungsbasis nach oben erstreckt, wobei der Säulenabschnitt eine Säuleneinstellvorrichtung (702) aufweist, die eingerichtet ist, um eine Höhe des Säulenabschnitts einzustellen;
- zumindest einen Arm (306), wobei der Arm eine Mehrzahl von Drehgelenken (314) aufweist und wobei ein proximaler Abschnitt (308) am zumindest einen Arm mit dem Säulenabschnitt über ein Drehgelenk verbunden ist; und
- einen Adapter (310), der mit einem distalen Abschnitt (312) am zumindest einen Arm über ein weiteres Drehgelenk verbunden ist, wobei der Adapter eingerichtet ist, um mit einer Patientenfixierungsvorrichtung (128) verbunden zu werden, die an einem anatomischen Merkmal (130) eines Patienten (118) befestigt ist, wobei das Patientenstabilisierungssystem eingerichtet ist, um die Patientenfixierungsvorrichtung in einem gesicherten Zustand an der Oberfläche zu verankern,
- wobei der Verriegelungsmechanismus eine Auslösevorrichtung (322) umfasst, um den Verriegelungsmechanismus (318) zwischen dem gesicherten Zustand und einem ungesicherten Zustand zu betätigen,
- wobei das chirurgische System (100) die Patientenfixierungsvorrichtung (128) aufweist und wobei die Stabilisierungsbasis (300), der Säulenabschnitt (302) und der Arm (306) zwischen der Oberfläche (136) und dem anatomischen Merkmal (130) des Patienten (118) positioniert sind.

2. Chirurgisches System nach Anspruch 1, wobei jedes Rad der Mehrzahl von Rädern in einem jeweiligen Radmodul (726) einer Mehrzahl von Radmodulen montiert ist und wobei die Mehrzahl von Radmodulen an der Stabilisierungsbasis montiert sind.

3. Chirurgisches System nach Anspruch 1, wobei die Mehrzahl von Rädern Omni-Räder (728) aufweist.

4. Chirurgisches System nach Anspruch 1, wobei der Verriegelungsmechanismus eingerichtet ist, um jedes Rad der Mehrzahl von Rädern in ein jeweiliges Radmodul im gesicherten Zustand zurückzuziehen.

5. Chirurgisches System nach Anspruch 1, wobei die Stabilisierungsbasis eine Mehrzahl von Stützstangen (436) aufweist, die sich von der Stabilisierungsbasis nach unten erstrecken, wobei die Mehrzahl von Stützstangen eingerichtet ist, um die Stabilisierungsbasis als Reaktion auf ein Zurückziehen jedes Rades der Mehrzahl von Rädern in ein entsprechendes Radmodul zu stützen.

6. Chirurgisches System nach Anspruch 5, wobei die Mehrzahl von Stützstangen drei Stützstangen aufweisen und wobei jede der drei Stützstangen in der Nähe eines anderen Rades der Mehrzahl von Rädern angeordnet ist.

7. Chirurgisches System nach Anspruch 5, wobei ein Kontakt zwischen der Mehrzahl von Stützstangen und der Oberfläche eingerichtet ist, um eine Bewegung der Stabilisierungsbasis in Bezug auf die Oberfläche einzuschränken.

8. Chirurgisches System nach Anspruch 1, wobei die Säuleneinstellvorrichtung eine Handkurbel (704) aufweist.

9. Chirurgisches System nach Anspruch 8, wobei die Handkurbel einen internen Leitspindelbaum (706) aufweist und wobei die interne Leitspindel eine Steigung aufweist, die eingerichtet ist, um ein Zurückdrehen der internen Leitspindel einzuschränken.

10. Chirurgisches System nach Anspruch 1, wobei zumindest eins der Drehgelenke ein Hirth-Gelenk aufweist.

11. Chirurgisches System nach Anspruch 1, wobei die Oberfläche einen Boden eines Operationssaals umfasst.

## Revendications

1. Système chirurgical (100) comprenant un système de stabilisation de patient (102), le système de stabilisation de patient comprend:
une base de stabilisation (300) ;
une pluralité de roues (316) montées sur la base de stabilisation, la pluralité de roues étant configurée pour déplacer la base de stabilisation le long d'une surface (136) ;
un mécanisme de verrouillage (318) configuré pour restreindre sélectivement le mouvement de la base de stabilisation par rapport à la surface ;
une partie de colonne (302) s'étendant vers le haut à partir de la base de stabilisation, la partie de colonne comprenant un dispositif de réglage de colonne (702) configuré pour régler la hauteur de la partie de colonne;
au moins un bras (306), dans lequel le bras comprend une pluralité d'articulations pivotantes (314), et dans lequel une partie proximale (308) de l'au moins un bras est couplée à la partie de colonne par l'intermédiaire d'une articulation pivotante; et
un adaptateur (310) couplé à une partie distale (312) de l'au moins un bras par l'intermédiaire d'une autre articulation pivotante, dans lequel l'adaptateur est configuré pour se coupler à un appareil de fixation du patient (128) fixé à une caractéristique anatomique (130) d'un patient (118), dans lequel le système de stabilisation du patient est configuré pour ancrer l'appareil de fixation du patient à la surface dans un état sécurisé,
le mécanisme de verrouillage comprend un dispositif de déclenchement (322) pour actionner le mécanisme de verrouillage (318) entre l'état sécurisé et l'état non sécurisé,
dans lequel le système chirurgical (100) comprend l'appareil de fixation du patient (128) et dans lequel la base de stabilisation (300), la partie de colonne (302) et le bras (306) sont positionnés entre la surface (136) et la caractéristique anatomique (130) du patient (118).

2. Système chirurgical selon la revendication 1, dans lequel chaque roue de la pluralité de roues est montée dans un module de roue respectif (726) d'une pluralité de modules de roue, et dans lequel la pluralité de modules de roue est montée sur la base de stabilisation.

3. Système chirurgical selon la revendication 1, dans lequel la pluralité de roues comprend des roues omni (728).

4. Système chirurgical selon la revendication 1, dans lequel le mécanisme de verrouillage est configuré pour rétracter chaque roue de la pluralité de roues dans un module de roue respectif à l'état sécurisé.

5. Système chirurgical selon la revendication 1, dans lequel la base de stabilisation comprend une pluralité de poteaux de support (436) s'étendant vers le bas à partir de la base de stabilisation, dans lequel la pluralité de poteaux de support est configurée pour soutenir la base de stabilisation en réponse à la rétraction de chaque roue de la pluralité de roues dans un module de roue respectif.

6. Système chirurgical selon la revendication 5, dans lequel la pluralité de poteaux de support comprend trois poteaux de support, et dans lequel chacun des trois poteaux de support est disposé à proximité d'une roue différente de la pluralité de roues.

7. Système chirurgical selon la revendication 5, dans lequel le contact entre la pluralité de poteaux de support et la surface est configuré pour limiter le mouvement de la base de stabilisation par rapport à la surface.

8. Système chirurgical selon la revendication 1, dans lequel le dispositif de réglage de colonne comprend une manivelle (704).

9. Système chirurgical selon la revendication 8, dans lequel la manivelle comprend une vis d'entraînement interne (706), et dans lequel la vis d'entraînement interne comprend un pas configuré pour restreindre l'entraînement arrière de la vis d'entraînement interne.

10. Système chirurgical selon la revendication 1, dans lequel l'au moins une des articulations pivotantes comprend un accouplement Hirth.

11. Système chirurgical selon la revendication 1, dans lequel la surface comprend le sol d'une salle d'opération.
